# EUROPEAN PATENT APPLICATION

(11) **EP 4 564 005 A1**
(43) Date of publication of application: **04.06.2025**
(21) Application number: 23383230.2
(22) Date of filing: 29.11.2023
(51) Int. Cl.: G01N 33/566, G01N 33/68

(54) **IN VITRO METHOD FOR DIAGNOSING OR SCREENING CHRONIC INFLAMMATORY DISEASES**

(71) Applicant: Airbiometrics Advanced Solutions SL, 12003 Castellón de la Plana (ES)
(72) Inventor: MACIAS CEJA, Dulce Carolina, 12003 Castellón de la Plana (ES); BARRACHINA SANCHO, Mª Dolores, 46010 Valencia (ES)
(74) Representative: Hoffmann Eitle

(57) **Abstract**

The present invention refers to an *in vitro* method for diagnosing or screening a chronic inflammatory disease selected from the group comprising: Inflammatory bowel disease (IBD), arthritis or psoriasis.

## Description

### FIELD OF THE INVENTION

The present invention refers to the medical field. Particularly, the present invention refers to an *in vitro* method for diagnosing or screening a chronic inflammatory disease selected from the group comprising: Inflammatory bowel disease (IBD), arthritis or psoriasis.

### STATE OF THE ART

Inflammation is a physiological response of the immune system to harmful stimuli, such as that the host enacts to defend itself against viruses, bacteria, tissue damage, metabolic stress by recruiting immune and non-immune cells. In some situations, this process becomes chronic, and may result in systemic effects beyond their primary target organs, underscoring the complex nature of chronic inflammatory diseases (CID). This term encompass a diverse group of medical conditions such as: asthma, chronic obstructive pulmonary disease, cancer, cardiovascular disease, neurological diseases like Parkinson's disease, systemic lupus erythematosus, rheumatoid arthritis, psoriasis and IBD.

IBD is a relapsing chronic disorder of the gastrointestinal tract associated with alteration in the epithelial barrier function and mucosal inflammation. The main idiopathic forms of IBD are Crohn's disease (CD) and ulcerative colitis (UC); both share some clinical and demographic characteristics but differ in the tissue damage they produce and their prognosis. CD can involve any part of the digestive tract and is characterized by transmural inflammation, which means it can affect multiple layers of the intestinal wall. UC, is characterized by inflammation and ulcerations that primarily affects the colon and rectum, causing inflammation limited to the mucosal layer. Rheumatoid Arthritis-Psoriasis (RAP) are two distinct autoimmune diseases. Rheumatoid arthritis (RA) specifically targets the joints, inducing chronic inflammation, joint pain, and potential deformities if not tackled. Additionally, RA can affect other organs, such as the heart and lungs. On the other hand, Psoriasis (P) represents a skin disorder that develops red, scaly patches on the skin, as a result of an overactive immune response. These conditions affect diverse bodily systems, yet they are linked by immune system dysfunction and persistent inflammation.

These pathologies share the pharmacological treatment used by clinicians to reduce inflammation, alleviate symptoms, and prevent or manage complications. One of the overarching goals in CID remains to accelerate its diagnosis to adapt treatment and improve its symptoms. In general, the diagnosis of chronic inflammatory diseases takes long time because it involves an evaluation that integrates a medical history, physical examinations, and various diagnostic tests which include specialized imaging techniques like X-rays and magnetic resonance imaging (MRI). Moreover, in the case of IBD, invasive procedure such as endoscopy and endoscopy and colonoscopy, often couple with tissue biopsies, may be required for a definitive diagnosis.

Consequently, there is an unmet medical need of finding reliable and non-invasive tools for the diagnosis or screening chronic inflammatory diseases like IBD, arthritis or psoriasis. The present invention is focused on solving this problem by the determination of inflammatory markers in saliva thus providing clinicians with a simple and rapid test which, combined with a thorough anamnesis allow them an early diagnosis of CID and a prompt treatment to control the symptoms.

### DESCRIPTION OF THE INVENTION

### Brief description of the invention

As indicated above, the present invention is directed to a reliable and non-invasive method for the diagnosis or screening chronic inflammatory diseases like IBD, arthritis or psoriasis by assessing specific biomarker in saliva.

So, the special technical feature conferring unity of invention to the present invention is the use of saliva samples for the diagnosis or screening chronic inflammatory diseases like IBD, arthritis or psoriasis by assessing specific biomarkers.

Such as it can be seen in the **Example 2,** protein levels and RNA expression of several specific biomarkers in saliva are associated with the presence of CID, such as IBD, Rheumatoid arthritis-psoriasis (RPA).

So, the first embodiment of the present invention refers to an *in vitro* method for identifying biomarker signatures for the diagnosis or screening of a chronic inflammatory disease selected from the group comprising or consisting of: IBD, arthritis or psoriasis in a subject, which comprises: a) Assessing the expression level of at least one protein selected from the group consisting of: IL1B and/or SIRT1, or of the genes encoding those proteins, in a saliva sample obtained from the subject, and b) wherein a deviation of the expression level of the proteins of step a) or of the genes encoding those proteins, as compared to a pre-established reference value, is an indication that the biomarker signature may be used for the diagnosis or screening of a chronic inflammatory disease selected from the group comprising or consisting of: IBD, arthritis or psoriasis.

In a preferred embodiment, the first embodiment of invention further comprises assessing the expression level of the protein calprotectin, or of the gene encoding this protein, in a saliva sample obtained from the subject.

The second embodiment of the present invention refers to an *in vitro* method for diagnosing or screening a chronic inflammatory disease selected from the group comprising or consisting of: IBD, arthritis or psoriasis in a subject, which comprises: a) Assessing the expression level of at least one protein selected from the group consisting of: IL1B and/or SIRT1, or of the genes encoding these proteins, in a saliva sample obtained from the subject, and b) wherein a higher expression level of the protein IL1B and/or a lower expression level of the protein SIRT1, or of the genes encoding those proteins, as compared to a pre-established reference value, is indicative that the subject suffers from IBD, arthritis and/or psoriasis.

In a preferred embodiment, the second embodiment invention further comprises assessing: a) Assessing the expression level of the protein calprotectin or of the gene encoding this protein, b) wherein a higher expression level of the protein calprotectin, or of the gene encoding that protein, as compared to a pre-established reference value, is indicative that the subject suffers from IBD, or b) wherein a higher expression level of the protein calprotectin, as compared to a pre-established reference value, is indicative that the subject suffers from arthritis or psoriasis, or c) wherein a lower abundance of mRNA transcripts encoding calprotectin, as compared to a pre-established reference value, is indicative that the subject suffers from arthritis or psoriasis.

In a preferred embodiment, the present invention comprises: a) Assessing the expression level of the proteins IL1B and calprotectin or of the gene encoding this protein, and b) wherein a higher expression level of the proteins IL1B and calprotectin, or of the gene encoding these proteins, as compared to a pre-established reference value, is indicative that the subject suffers from IBD.

In a preferred embodiment, the present invention further comprises: a) Assessing the expression level of the protein SIRT1 or of the gene encoding this protein, and b) wherein a decreased expression level of the protein SIRT1, or of the gene encoding that protein, as compared to a pre-established reference value, is indicative that the subject suffers from IBD.

In a preferred embodiment, the IBD disease is selected from: Chron's disease or ulcerative colitis.

The third embodiment of the present invention refers to the *in vitro* use of the proteins IL1B and/or SIRT1, or of the transcripts encoding thereof, or of a kit comprising reagents for the identification of the proteins IL1B and/or SIRT1, or of the transcripts encoding thereof, for identifying biomarker signatures for the diagnosis or screening of a chronic inflammatory disease selected from the group comprising: IBD, arthritis or psoriasis, wherein the IBD is preferably selected from Chron's disease or ulcerative colitis.

In a preferred embodiment, the present invention refers to the *in vitro* use of the proteins IL1B and/or SIRT1, or of the transcripts encoding thereof, in combination with the protein calprotectin, or of the transcripts encoding thereof, or of a kit comprising reagents for the identification of the proteins IL1B and/or SIRT1, or of the transcripts encoding thereof, in combination with calprotectin, or of the transcripts encoding thereof.

In a preferred embodiment, the present invention refers to the *in vitro* use of the proteins IL1B and/or SIRT1, or of the transcripts encoding thereof, or of a kit comprising reagents for the identification of the proteins IL1B and/or SIRT1, or of the transcripts encoding thereof, for the diagnosis or screening of a chronic inflammatory disease selected from the group comprising: IBD, arthritis or psoriasis, wherein the IBD is preferably selected from Chron's disease or ulcerative colitis.

In a preferred embodiment, the present invention refers to the *in vitro* use of the proteins IL1B and/or SIRT1, or of the transcripts encoding thereof, in combination with calprotectin, or of a kit comprising reagents for the identification of the proteins IL1B and/or SIRT1, or of the transcripts encoding thereof, in combination with calprotectin.

In a preferred embodiment, the present invention refers to the *in vitro* use of proteins IL1B and calprotectin, or of the transcripts encoding thereof, or of a kit comprising reagents for the identification of the proteins IL1B and calprotectin, or of the transcripts encoding thereof, for the diagnosis or screening IBD, preferably selected from Chron's disease or ulcerative colitis.

In a preferred embodiment, the present invention refers to the *in vitro* use of the proteins IL1B, calprotectin and SIRT1, or of the transcripts encoding thereof, or of a kit comprising reagents for the identification of the proteins IL1B, calprotectin and SIRT1, or of the transcripts encoding thereof.

In a preferred embodiment, the present invention refers to the *in vitro* use of biomarker signatures identified by following the method of the invention, for the diagnosis or screening of a chronic inflammatory disease selected from the group comprises: IBD, arthritis or psoriasis, wherein the IBD is preferably selected from Chron's disease or ulcerative colitis.

In a preferred embodiment, the present invention refers to anti-inflammatory drugs (see **Table 1**), preferably selected from the group comprising: a) salicylates; b) immunosuppressants selected from azathioprine, methotrexate and mercaptopurine; c) biological treatments selected from adalimumab, golimumab, infliximab, risankisumab, upadicitinib, ustekinumab and vedolizumab; or d) corticosteroids selected from: prednisone, cortisone, betamethasone, budesonide and beclomethasone; for use in the treatment of a chronic inflammatory disease selected from the group comprising: IBD, preferably selected from Chron's disease or ulcerative colitis, arthritis or psoriasis, wherein the method comprises diagnosing patients that suffer from the chronic inflammatory disease by using the signatures identified by following the method of the invention.

**Table 1: Pharmacological treatment used for chronic inflammatory diseases.**

| Salicylates | Mesalazine |
|---|---|
| Immunosuppressants | Azathioprine, Methotrexate, Mercaptopurine |
| Biological treatment | Adalimumab, Golimumab, Infliximab, Risankisumab, Upadicitinib, Ustekinumab, Vedolizumab |
| Corticosteroids | Prednisone, Cortisone, Betamethasone, Budesonide, and Beclomethasone (Clipper) |
| Combined treatment | Immunosuppressive and biological |

The present invention also refers to an *in vitro* method for identifying biomarker signatures for the diagnosis or screening of a chronic inflammatory disease selected from the group comprising: IBD, arthritis or psoriasis in a subject, which comprises: a) Assessing the expression level of at least one protein selected from the group consisting of: IL1B, SIRT1 and/or calprotectin, or of the genes encoding those proteins, in a saliva sample obtained from the subject, and b) wherein a deviation of the expression level of the proteins of step a) or of the genes encoding those proteins, as compared to a pre-established reference value, is an indication that the subject suffers from: IBD, arthritis or psoriasis.

The present invention refers to an *in vitro* method for the differential diagnosis between active and remissive disease in a subject suffering from IBD, which comprises: a) Assessing the expression level of at least one protein selected from the group consisting of: IL1B and/or calprotectin, or of the genes encoding those proteins, in a saliva sample obtained from the subject, b) wherein a higher expression level of the protein IL1B, or of the gene encoding thereof, as compared to a pre-established reference value, is indicative that the subject suffers from active IBD, or c) wherein a higher abundance of mRNA transcripts encoding calprotectin, as compared to a pre-established reference value, is indicative that the subject suffers from IBD, or d) wherein a lower expression level of the protein IL1B, or of the gene encoding thereof, as compared to a pre-established reference value, is indicative that the subject suffers from remissive IBD, or e) wherein a lower abundance of mRNA transcripts encoding calprotectin, as compared to a pre-established reference value, is indicative that the subject suffers from remissive IBD.

The present invention also refers to an *in vitro* method for the differential diagnosis between Chron's disease and ulcerative colitis in a subject suffering from IBD, which comprises: a) Assessing the expression level of the protein SIRT1, or of the genes encoding thereof, in a saliva sample obtained from the subject, b) wherein a higher expression level of the protein SIRT1, or of the gene encoding thereof, as compared to a pre-established reference value, is indicative that the subject suffers from Chron's disease, or c) wherein a lower expression level of the protein SIRT1, or of the gene encoding thereof, as compared to a pre-established reference value, is indicative that the subject suffers from ulcerative colitis.

In a preferred embodiment, the chronic inflammatory disease, IBD, psoriasis or arthritis is active or remissive.

In a preferred embodiment, the subject is a subject suffering from IBD that has received a treatment selected from: corticosteroids, biological or immunosuppressants.

In a preferred embodiment, the IBD is selected from: Chron's disease or ulcerative colitis.

On the other hand, the present invention also refers to:
A method for detecting a biomarker in a saliva sample from a human subject at risk of developing a chronic inflammatory disease selected from the group comprising: IBD, arthritis or psoriasis, the method comprising performing an assay for determining the concentration of the protein or level of expression of the genes of the above cited biomarkers, such as for instance, an immunoassay or PCR.

In a preferred embodiment, the present invention is a computer-implemented invention, wherein a processing unit (hardware) and a software are configured to: a) Receive the concentration level or level of expression values of any of the above cited biomarkers or signatures, b) process the concentration level values received for finding substantial variations or deviations, and c) provide an output through a terminal display of the variation or deviation of the concentration or expression level, wherein the variation or deviation of the concentration or expression level indicates that the subject may be suffering from a chronic inflammatory disease selected from the group comprising: IBD, arthritis or psoriasis.

The present invention also refers to a computer program or a computer-readable media containing means for carrying out the present invention.

For the purpose of the present invention, the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of" indicates that the listed elements are required or mandatory, and that no other elements may be present.
- According to the present invention, a reference value can be a "pre-established threshold value" or a "cut-off' value. Typically, a "threshold value" or "cut-off value" can be determined experimentally, empirically, or theoretically. According to the present invention, the "pre-established threshold" value refers to a value previously determined in control subjects who are nonsuffering from a chronic inflammatory disease selected from the group comprising: IBD, arthritis or psoriasis. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Brief description of the figures

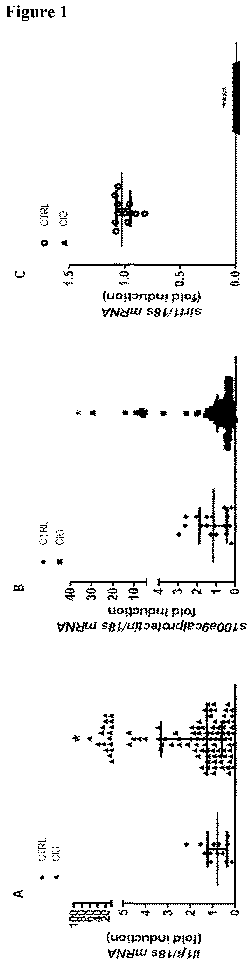

**Figure 1****.** Increased mRNA expression of inflammatory biomarkers in saliva of chronic inflammatory disease (CID) patients. **A)** mRNA expression of Il1β, **B)** mRNA expression of s100s9 calprotectin and **C)** mRNA expression sirt1 in saliva of CID patients and control samples. Data show median and the interquartile range of gene expression; each dot represents the value of an individual expressed as fold induction vs the mean of control samples Mann-Whitney U test was used and *P < 0.05 indicate the statistical significance vs controls.

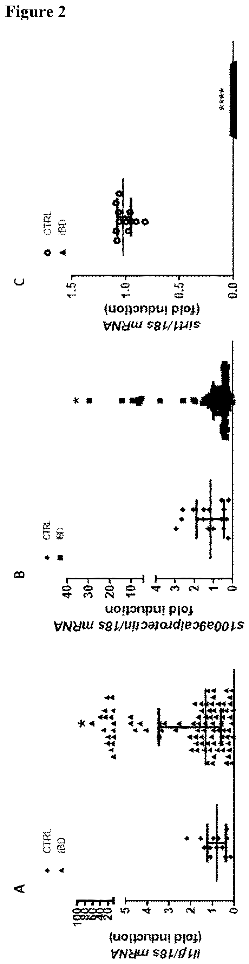

**Figure 2****.** Increased mRNA expression of inflammatory biomarkers in saliva of inflammatory bowel disease (IBD) patients. **A)** mRNA expression of 1110, **B)** mRNA expression of s100a9 calprotectin and **C)** mRNA expression sirt1 in saliva of IBD patients and control samples. Data show median and the interquartile range of gene expression; each dot represents the value of an individual expressed as fold induction vs the mean of control samples. Mann-Whitney U test was used and *P < 0.05 indicate the statistical significance vs controls.

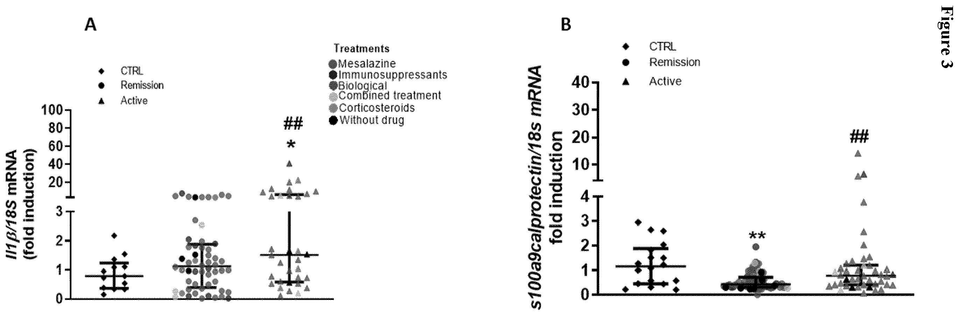

**Figure 3****.** Increased mRNA expression of inflammatory biomarkers in saliva of active vs remission IBD patients. **A)** mRNA expression of Il1β and **B)** mRNA expression of s100a9 calprotectin in saliva from both remission and active IBD patients and healthy controls. Data show median and the interquartile range of gene expression; each dot represents the value of an individual expressed as fold induction vs the mean of control samples. The colors represent treatments received by of patients: pink (mesalazine), blue (Immunosuppressant), red (biological), sky blue (combined treatment), green (corticosteroids) and black (without drug). Mann-Whitney U test and one-way ANOVA were used and *P<0.05, **P<0.01 and ***P < 0.001 indicate the statistical significance vs controls while ##P<0.01 vs IBD patients in remission. Mann-Whitney U test.

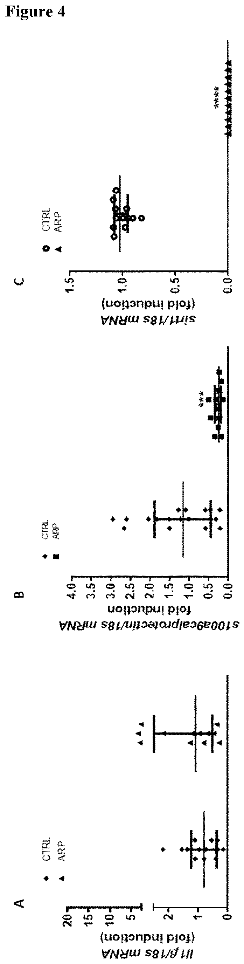

**Figure 4****.** mRNA expression of inflammatory biomarkers in rheumatoid arthritis-psoriasis (ARP) patients. **A)** mRNA expression of Il1β, **B)** mRNA expression of s100a9 calprotectin and C) mRNA expression of sirt1 in saliva of ARP patients and control samples. Data show median and the interquartile range of gene expression; each dot represents the value of an individual expressed as fold induction vs the mean of control samples. Mann-Whitney U test was used and ***P < 0.001 indicate the statistical significance vs controls.

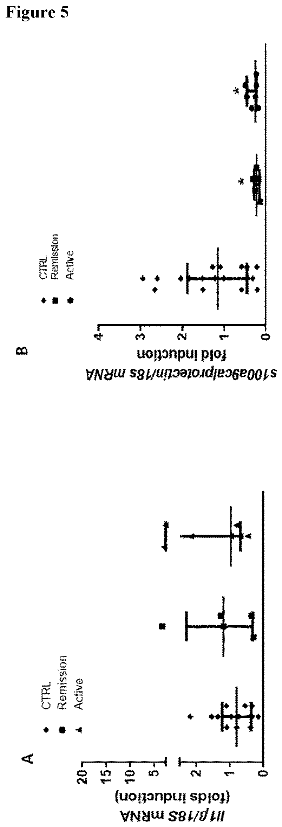

**Figure 5****.** mRNA expression of inflammatory biomarkers in saliva of both, active and remission rheumatoid arthritis-psoriasis (ARP) patients. **A)** mRNA expression of Il1β and **B)** mRNA expression of s100a9 calprotectin in saliva from both remission and active ARP patients and healthy controls. Data show median and the interquartile range of gene expression; each dot represents the value of an individual expressed as fold induction vs the mean of control samples. Mann-Whitney U test and one-way ANOVA were used and *P<0.05, indicate the statistical significance vs controls.

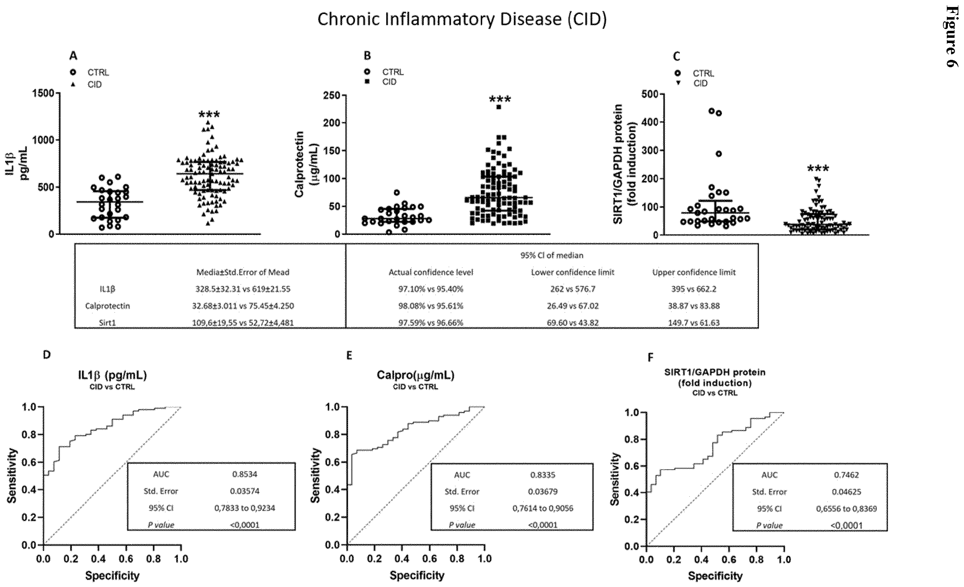

**Figure 6****.** Increased protein expression of Il1β, calprotectin and SIRT1 in chronic inflammatory disease (CID) patients. **A, B, C)** Area under the receiver operating characteristic (ROC) curve of IL1β, calprotectin and SIRT1, respectively in saliva to discriminate between individuals with CID patients and control subjects. **D, E, F)** Protein expression levels in saliva of CID patients and control individuals of IL1β, calprotectin and SIRT1, respectively. Data show median and the interquartile range of gene expression; each dot represents the value of an individual expressed as fold induction vs the mean of control samples. Mann-Whitney U test and one-way ANOVA were used and *P<0.05 and ***P<0.001 indicate the statistical significance vs controls.

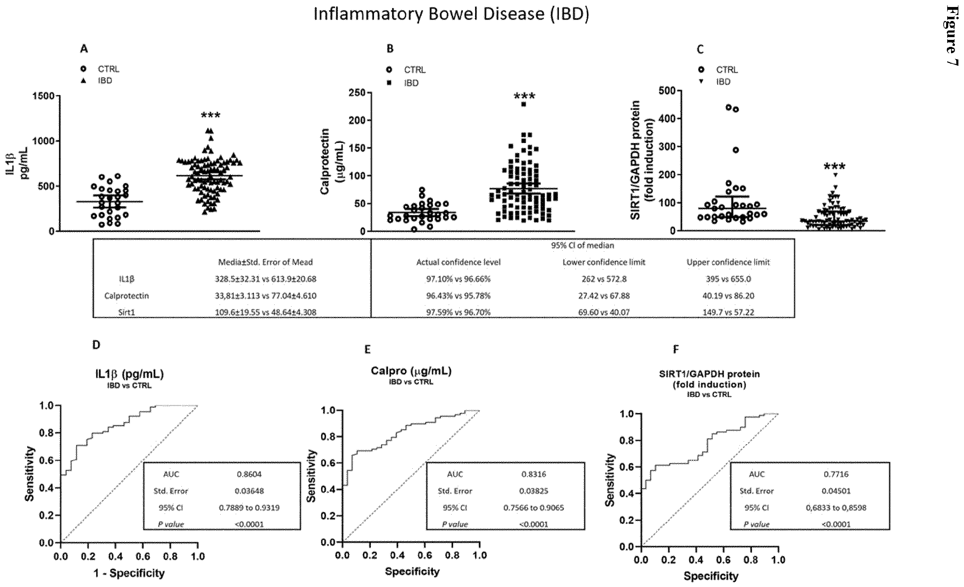

**Figure 7****.** Increased protein expression of IL1β, calprotectin and SIRT1 in inflammatory bowel disease (IBD) patients. **A, B, C)** Area under the receiver operating characteristic (ROC) curve of IL1β, calprotectin and SIRT1, respectively in saliva to discriminate between individuals with IBD patients and control subjects. **D, E, F)** Protein expression levels in saliva of IBD patients and control individuals of IL1β, calprotectin and SIRT1, respectively. Data show median and the interquartile range of gene expression; each dot represents the value of an individual expressed as fold induction vs the mean of control samples. Mann-Whitney U test and one-way ANOVA were used and **P<0.01 and ***P<0.001 indicate the statistical significance vs controls.

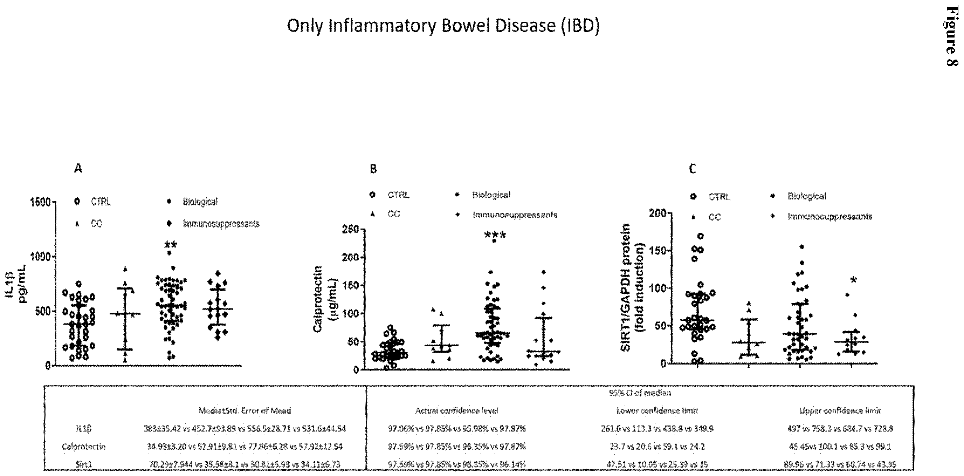

**Figure 8****.** Comparison of salivary concentrations of biomarkers in saliva of inflammatory bowel disease (IBD) patients depending on the pharmacological treatment received. Graphs show protein levels of **A)** IL1β, **B)** calprotectin and C) SIRT1 levels in saliva of control subjects and IBD patients classified according to the pharmacological treatment received (CC: corticosteroids, biological and immunosuppressors). Data show median and the interquartile range of gene expression; each dot represents the value of an individual expressed as fold induction vs the mean of control samples. Mann-Whitney U test and one-way ANOVA were used and *P<0.05, **P<0.01 and ***P<0.001 indicate the statistical significance vs controls.

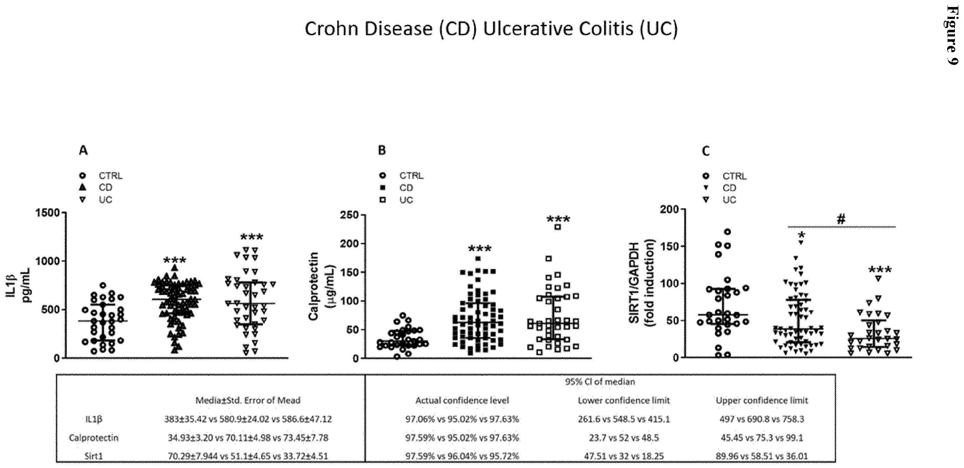

**Figure 9****.** Analysis of IL1β, calprotectin and SIRT1 protein levels in Crohn disease (CD) and Ulcerative colitis (UC) patients. Graphs show protein levels of **A)** IL1β, **B)** calprotectin and C) SIRT1 levels in saliva of control subjects and patients with Crohn's disease (CD) or Ulcerative colitis (UC). Data show median and the interquartile range of gene expression; each dot represents the value of an individual expressed as fold induction vs the mean of control samples. Mann-Whitney U test and one-way ANOVA were used and *P<0.05 and ***P<0.001 indicate the statistical significance vs controls and #P<0.05 vs CD.

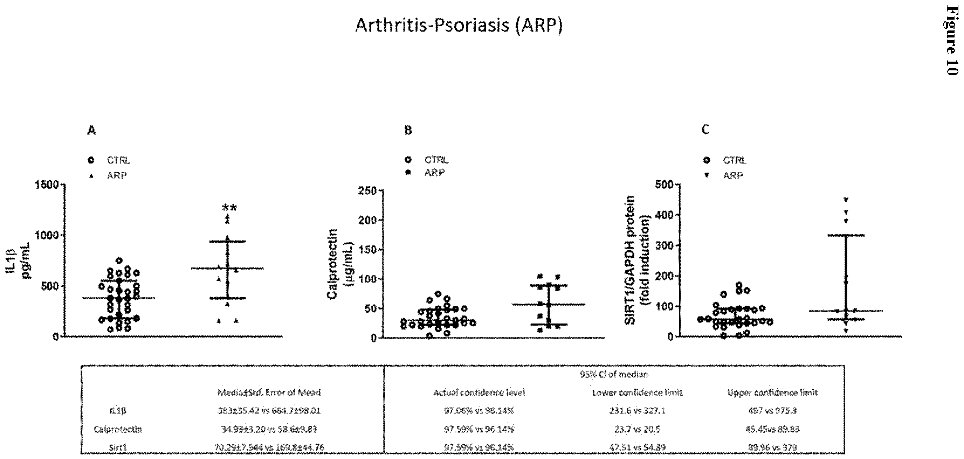

**Figure 10****.** Protein expression of inflammatory biomarkers in rheumatoid arthritis-psoriasis (ARP) patients. Protein expression of **A)** IL1β, **B)** S100A9 calprotectin and C) SIRT-1 in saliva of ARP patients and control samples. Data show median and the interquartile range of gene expression; each dot represents the value of an individual expressed as fold induction vs the mean of control samples. Mann-Whitney U test was used and **P < 0.01 indicate the statistical significance vs controls.

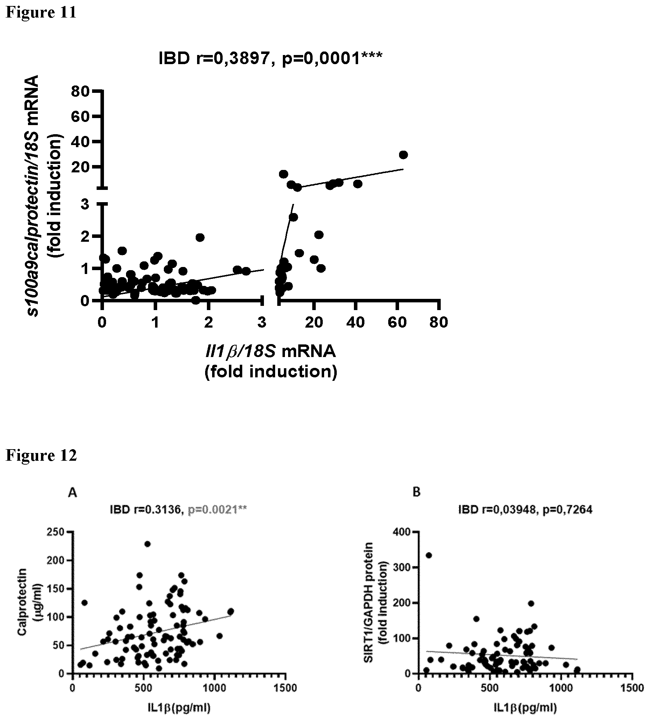

**Figure 11****.** Correlation between the mRNA expression levels of IL1β and S100a9 calprotectin in saliva of IBD patients. A positive and significant correlation is detected between the mRNA expression of S100a9 calprotectin and IL1β (expressed as fold induction) in saliva of IBD patients. The Spearman's correlation coefficient (R) and p value are also shown.
**Figure 12****.** Correlation between protein levels of IL1β and S100a9 calprotectin as well as IL1b and SIRT1 in saliva of IBD patients. **A)** A positive and significant correlation is detected between the mRNA expression of S100a9 calprotectin and IL1β (expressed as fold induction) in saliva of IBD patients. **B)** A positive and no significant correlation is detected between the mRNA expression of SIRT1 and IL1β (expressed as fold induction) in saliva of IBD patients The Spearman's correlation coefficient (R) and p value are also shown.

### Detailed description of the invention

The present invention is illustrated by means of the Examples set below without the intention of limiting its scope of protection.

### Example 1. Material and methods

### Example 1.1. Patients

A bulk saliva sample from all control and patients included in Table 1 was obtained. Control patients were healthy subjects who fail to suffer any inflammatory pathology. IBD patients were classified by healthcare professional as active (those who present >200µg/ml calprotectin in feces) or remission (those who present <200µg/ml calprotectin in feces). Rheumatoid arthritis and Psoriasis patients were classified between active or remission according with the symptoms they present inflammation joint deformity articular and psoriatic plaque. The information on all the patients analysed in this study is summarized in **Table 2.** The study was approved by the Institutional Review Board of the University Clinical Hospital (Valencia, Spain). Written informed consent was obtained from all participating patients.

**Table 2**

| | **Controls** | **Inflammatory Bowel Disease** | **Rheumatoid arthritis (AR)** | **Psoriasis (P)** |
|---|---|---|---|---|
| **Number of patients** | 30 | 102 | 5 | 7 |
| **Age (y)** | | | | |
| **18-40 years** | 23 | 46 | 1 | 6 |
| **>40 years** | 7 | 58 | 4 | 1 |
| **Idiopathic** | | CD=65 and UC=37 | | |
| **Clinical Activity** | | Remission=62 Active=40 | Remission=2 Active=3 | Remission=3 Active=4 |
| **Gender** | | | | |
| **Male** | 11 | 56 | 4 | 4 |
| **Female** | 19 | 46 | 1 | 3 |

### Example 1.2. RNA isolation and Real-Time Quantitative PCR (RT-pPCR)

Total RNA from saliva was isolated using direct RNAspin 96 isolation kit from Cytiva Amersham^{™} according to the manufacturer's instructions. cDNA was obtained from previously isolated RNA by reverse transcription PCR using the High-capacity cDNA RT reagent Kit (appliedbiosystems, Baltics, UAB). Gene expression was analyzed by real-time Quantitative PCR using SYBR^{®} Ex Taq (Takara Bio Inc., Saint-Germain-en-Laye, France) in LightCycler thermocycler (Roche Diagnostics, Mannheim, Germany). Specific oligonucleotides detailed in table 2 were designed to perform the analysis. The relative gene expression, as fold increase vs control, was expressed as follows: change in expression (fold) = 2 - Δ(ΔCT) where ΔCT = CT (target) - CT (housekeeping) and Δ(ΔCT) = ΔCT (treated) - ΔCT (control), where β-actin was the housekeeping gene used.

**Table 3**

| **Gene** | **Sense (5'-3')** | **Antisense (3'-5')** | **Fragment 's Size (bp)** |
|---|---|---|---|
| *Il1B* | TTTTTGCTGTGAGTCCCG GAG (SEQ ID NO: 1) | TTTTTGCTGTGAGTCCCG GAG (SEQ ID NO: 2) | 84 |
| *s100a9* | AGTGGCCAAGATCACAG TGG (SEQ ID NO: 3) | GCCTGGCCTCCTGATTAG TG (SEQ ID NO: 4) | 80 |
| *sirt1* | TCCAAGTTCCATACCCCA TGA (SEQ ID NO: 5) | CAAAATGCAGATGAGGC AAAG (SEQ ID NO: 6) | 71 |
| *18s* | CGGCTACCACATCCAAG GAA (SEQ ID NO: 7) | GTCGGAATTACCGCGGC T (SEQ ID NO: 8) | 186 |

*Sequences of human primers used in real-time PCR*

### Example 1.3. Protein quantification and Western blot Analysis

Protein was isolated from bulk saliva. Western Blot was performed to analyze protein expression. SDS-PAGE gels were used, and equal amounts of protein were loaded. Then, proteins were transferred to nitrocellulose membranes, which were further incubated with specific primary antibodies (detailed in table 3 as well as the secondary antibodies peroxidase-conjugated anti-rabbit IgG (Thermo Scientific, Waltham, MA, USA, 1:5000). Protein bands were detected with Immobilon^{®} West Femto Western HRP Substrate (Thermofisher Scientific Vienna, Austria) in AMERSHAM ImageQuant 800 (GE lifescience, Cornellà de Llobregat, Spain). To normalize protein bands, Glyceraldehyde 3-phosphate dehydrogenase (GAPDH) was used as housekeeping. The densitometry of the bands was quantified using the software ImageJ.

**Table 4**

| **Antibody** | **Supplier** | **Dilution** |
|---|---|---|
| SIRT1 | AB32441, ABCAM | 1:1000 |
| GAPDH | G9545, Sigma-Aldrich | 1:2000 |

*Primary antibodies used for Western blot analysis*

### Example 1.4. ELISA

### Example 1.4.1 IL1β

Secreted protein levels of IL-1β from bulk saliva were quantified by ELISA using the human IL-1β high sensitivity ELISA KIT (Invitrogen, Thermofisher Scientific Vienna, Autria) following manufacturer's instructions. Bulk saliva sample of IBD-RAP patients and healthy controls were diluted 1:50 with PBS, then biotin conjugate is added to all wells. Samples were incubated the dark overnight at room temperature in the precoated 96-well strip plate. After, streptavidin-HRP was added during 1 hour at 18-25 °C. Then amplification solution I was incubated for 15 min at 18-25 °C and that, amplification solution II was then added at 18-25 °C during 30 min. TMB Substrate Solution was then added at 18-25 °C during 10-20 min. Finally, Stop Solution was added, and absorbance was measured at 450 nm with the microplate reader SpectaMax Plus 384 (Molecular Devices, San Jose, CA, USA).

### Example 1.4.2. Calprotectin

Secreted protein levels of calprotectin from bulk saliva were quantified by ELISA using the human calprotectin L1/S100-A8/A9 complex (Invitrogen, Thermofisher Scientific Vienna, Autria) following manufacturer's instructions. Bulk saliva sample of IBD-RAP patients and healthy controls were diluted 1:20000 with PBS. Samples were incubated 2.5 hours at room temperature in the precoated 96-well strip plate. Then, biotin conjugate is added to all was then added a room temperature for 1 hour and that, streptavidin-HRP was added during 45 min at room temperature. TMB Substrate Solution was then added at room temperature during 30 min. Finally, Stop Solution was added, and absorbance was measured at 450 nm with the microplate reader SpectaMax Plus 384 (Molecular Devices, San Jose, CA, USA).

### Example 1.5. Statistical Analysis

Data were represented as individual values and median ± interquartile range were analysed using GraphPad Prism V.8 San Diego, CA, USA and were compared by a t-test for comparisons between two groups with Mann-Whitney *U* test post hoc correction was employed for clinical data analysis by analysis of variance (one-way ANOVA) with Tukey post hoc correction where appropriate for multiple comparisons. Statistical significance was considered with a *p*-value < 0.05. Correlations from data obtained were analysed using Spearman's correlation coefficient.

### Example 2. Results

### Example 2.1. mRNA expression of inflammatory biomarkers in saliva

### Example 2.1.1. Chronic inflammatory disease (CID) patients

Our study aim to analyse the mRNA expression of pro-inflammatory markers in saliva of control subjects and CID patients, which include inflammatory bowel diseases (IBD) and rheumatoid arthritis-psoriasis (RPA) patients. The analysis of the mRNA expression of *Il1β* and *s100a9* calprotectin revealed a significant increase in the expression of these genes, in CID compared with levels detected in controls subjects (**Fig.1A****, B**). As show in **Fig. 1C****,** it is observed a decrease in the expression of *sirt1* in CID patients compared to controles subjects.

### Example 2.1.2. Inflammatory bowel diseases (IBD) patients

Next, we classified patients according with the specific pathology and results reveal a significant increase in the mRNA expression of both *Il1β* and *s100a9* calprotectin (**Fig.2A****, B**) in saliva from IBD patients compared with that of controls individuals. Different investigations indicate that the *sirt1* molecule is involved in different biological processes, including inflammation, and its main function is to block the NFκB factor. The results show that patients with IBD experience a decrease in SIRT1 mRNA expression levels compared with levels detected in controls subjects (**Fig.2C**).

### Example 2.1.3. Active vs remission Inflammatory bowel diseases (IBD) patients

Next, we classified IBD patients in those that had active disease and those who were in clinical remission, according with the clinical criteria and results are shown in different colours, depending on the pharmacological treatment received by patients. As shown in **Fig. 3****,** IBD patients in remission exhibited *Il1β* mRNA levels non-significantly different to those detected in control samples while levels of this cytokine in active IBD were significantly higher than both, those obtained in patients with clinical remission and those obtained in control subjects. In relation to the mRNA expression of *s100a9* calprotectin, levels in IBD patients in remission were significantly lower than those in control patients while levels in active IBD were significantly higher than those in patients with clinical remission. In all cases data show that the drug does not modulate the expression of these molecules.

### Example 2.1.4. Rheumatoid arthritis-psoriasis (RPA) patients

The reduced number of samples obtained from patients with arthritis or psoriasis led us to analyse samples from all of them together (RPA), and results show a non-significant increase in the mRNA expression of *Il1β* in patients compared with healthy controls (**Fig.4A**). In contrast, the mRNA expression of *s100a9* calprotectin and sirt1 was significantly decreased in saliva from RPA patients compared with that of control subjects (**Fig.4B****, C**).

### Example 2.1.5. Active vs remission Rheumatoid arthritis-psoriasis (RPA) patients

As shown in **Fig 5A****,** the mRNA expression of *Il1β* did not significantly differ between control subjects and patients with ARP, irrespective of the activity or the remission of the disease. In contrast, the mRNA expression of *s100a9* calprotectin (**Fig. 5B**) was significantly reduced in both remission and active ARP patients, compared with levels in control samples.

### Example 3. Protein expression of inflammatory biomarkers in saliva

### Example 3.1. Chronic inflammatory disease (CID) patients

The analysed the protein levels of IL1β, calprotectin and SIRT1 in saliva samples. As show in results, protein expression of IL1β and calprotectin (**Fig. 6A****, B**) was significantly increase in CID patients than in control subject. In addition, the level of Sirt1 was significantly lower in samples of CID patients compared with levels detected in controls subjects (**Fig. 6C**).

Subsequently, we analyse the ROC curve of these proteins in samples and results show proteins expression was analysed ROC curve, CID patients salivary IL1β (AUC-ROC = 0.8534) and calprotectin (AUC-ROC = 0.8335) had the highest AU-ROC for distinguishing patients with IBD from control individuals (**Fig. 6D****, E**). Supporting this result, we observed that the Sirt1 protein (AUC-ROC = 0.7462) (**Fig.6F**), which has an important exalt role in regulating inflammatory processes by inhibiting NPκB.

### Example 3.2. Inflammatory bowel disease (IBD) patients

Next, we classified patients according with the pathology and results reveal an increase in protein expression of both, IL1β and calprotectin in saliva of IBD patients when compared with levels in control samples (**Fig. 7A****, B**). Unlike these, Sirt1 shows levels lower in IBD group from control individuals (**Fig. 7C**).

Subsequently, proteins expression was analysed ROC curve, IBD patients salivary IL1β (AUC-ROC = 0.8604) and calprotectin (AUC-ROC = 0.8316) had the highest AU-ROC for distinguishing patients with IBD from control individuals (**Fig. 7D****, E**). Supporting this result, we have la sirt1 protein (AUC-ROC = 0.7716) (**Fig.7F**).

### Example 3.3. Protein expression of salivary biomarkers depending on the pharmacological treatment received by IBD patients

IBD patients were classified according to the pharmacological treatment received (**Fig.8**) and results show that patients receiving biological treatment exhibited a significant increase in the protein expression of the cytokine IL1β and calprotectin, compared with control samples while non-significant differences were detected in the expression of Sirt1. In contrast, patients receiving corticosteroids exhibited protein levels of IL1β, calprotectin and Sirt1 that were non-significantly different to that detected in control subjects. Finally, in receiving immunosuppressors protein levels of IL1β and calprotectin were non-significantly different than those obtained in control samples while Sirt1 levels were significantly lower.

### Example 3.4. Protein expression of salivary biomarkers in Crohn's disease and Ulcerative colitis

Finally, we classified IBD patients according to the specific clinical entity, Crohn's disease and Ulcerative Colitis. Results show a significant increase in protein levels of IL1β, and calprotectin while significant lower levels of Sirt1 in saliva of CD patients compared with control samples (**Fig. 9A****, B, C**). We also detected a significant increase in the protein levels of both IL1β and calprotectin in samples from UC patients compared with control samples while a significant diminution of Sirt1 levels was observed in UC patients compared with both control and CD patients (**Fig 9A****, B, C**).

### Example 3.4.1. Rheumatoid arthritis-psoriasis (RPA) patients

Additionally, when analysed protein expression in ARP patients results show higher levels of IL1β in ARP than in control group (**Fig.10A**). However, the protein expression of both calprotectin and Sirt1, was not significant different between ARP patients and control subjects (**Fig.10** **B, C**).

### Example 4. Correlation of inflammatory biomarkers in saliva of IBD patients

Data show a positive and significant correlation, by the Spearman rank test, between the mRNA of *IL1β* and the mRNA expression of S100A9 calprotectin in saliva of IBD patients (r = 0.4061, P = <0,0001) (**Fig. 11**).

The analysis of the correlation between protein levels of IL1β and S100A9 calprotectin also reveal a positive and significant correlation (**Fig. 12A**). Finally, protein levels of IL1β and SIRT1 also exhibited a positive no significant correlation (**Fig. 12B**).

## Claims

1. *In vitro* method for identifying biomarker signatures for the diagnosis or screening of a chronic inflammatory disease selected from the group comprising: inflammatory bowel disease (IBD), arthritis or psoriasis in a subject, which comprises:
a) assessing the expression level of at least one protein selected from the group consisting of: IL1B and/or SIRT1, or of the genes encoding those proteins, in a saliva sample obtained from the subject,
b) wherein a deviation of the expression level of the proteins of step a) or of the genes encoding those proteins, as compared to a pre-established reference value, is an indication that the biomarker signature may be used for the diagnosis or screening of a chronic inflammatory disease selected from the group comprising: IBD, arthritis or psoriasis.

2. *In vitro* method, according to claim 1, wherein step a) further comprises assessing the expression level of the protein calprotectin, or of the gene encoding this protein, in a saliva sample obtained from the subject.

3. *In vitro* method, for diagnosing or screening a chronic inflammatory disease selected from the group comprising: IBD, arthritis or psoriasis in a subject, which comprises:
a) assessing the expression level of at least one protein selected from the group consisting of: IL1B and/or SIRT1, or of the genes encoding these proteins, in a saliva sample obtained from the subject,
b) wherein a higher expression level of the protein IL1B and/or a lower expression level of the protein SIRT1, or of the genes encoding those proteins, as compared to a pre-established reference value, is indicative that the subject suffers from IBD, arthritis and/or psoriasis.

4. *In vitro* method, according to claim 3, which further comprises:
a) assessing the expression level of the protein calprotectin or of the gene encoding this protein,
b) wherein a higher expression level of the protein calprotectin, or of the gene encoding that protein, as compared to a pre-established reference value, is indicative that the subject suffers from IBD, or
c) wherein a higher expression level of the protein calprotectin, as compared to a pre-established reference value, is indicative that the subject suffers from arthritis or psoriasis, or
d) wherein a lower abundance of mRNA transcripts encoding calprotectin, as compared to a pre-established reference value, is indicative that the subject suffers from arthritis or psoriasis.

5. *In vitro* method, according to claim 3, for diagnosing or screening IBD in a subject, which comprises:
a) assessing the expression level of the proteins IL1B and calprotectin or of the gene encoding this protein,
b) wherein a higher expression level of the proteins IL1B and calprotectin, or of the gene encoding these proteins, as compared to a pre-established reference value, is indicative that the subject suffers from IBD.

6. *In vitro* method, according to claim 5, which further comprises:
a) assessing the expression level of the protein SIRT1 or of the gene encoding this protein,
b) wherein a decreased expression level of the protein SIRT1, or of the gene encoding that protein, as compared to a pre-established reference value, is indicative that the subject suffers from IBD.

7. *In vitro* method, according to any of the previous claims, wherein the IBD disease is selected from: Chron's disease or ulcerative colitis.

8. *In vitro* use of the proteins IL1B and/or SIRT1, or of the transcripts encoding thereof, or of a kit comprising reagents for the identification of the proteins IL1B and/or SIRT1, or of the transcripts encoding thereof, for identifying biomarker signatures for the diagnosis or screening of a chronic inflammatory disease selected from the group comprising: IBD, arthritis or psoriasis, wherein the IBD is preferably selected from Chron's disease or ulcerative colitis.

9. *In vitro* use, according to claim 8, of the proteins IL1B and/or SIRT1, or of the transcripts encoding thereof, in combination with the protein calprotectin, or of the transcripts encoding thereof, or of a kit comprising reagents for the identification of the proteins IL1B and/or SIRT1, or of the transcripts encoding thereof, in combination with calprotectin, or of the transcripts encoding thereof.

10. *In vitro* use of the proteins IL1B and/or SIRT1, or of the transcripts encoding thereof, or of a kit comprising reagents for the identification of the proteins IL1B and/or SIRT1, or of the transcripts encoding thereof, for the diagnosis or screening of a chronic inflammatory disease selected from the group comprising: IBD, arthritis or psoriasis, wherein the IBD is preferably selected from Chron's disease or ulcerative colitis.

11. *In vitro* use, according to claim 10, of the proteins IL1B and/or SIRT1, or of the transcripts encoding thereof, in combination with calprotectin, or of a kit comprising reagents for the identification of the proteins IL1B and/or SIRT1, or of the transcripts encoding thereof, in combination with calprotectin.

12. *In vitro* use, according to claims 10, of the proteins IL1B and calprotectin, or of the transcripts encoding thereof, or of a kit comprising reagents for the identification of the proteins IL1B and calprotectin, or of the transcripts encoding thereof, for the diagnosis or screening IBD, preferably selected from Chron's disease or ulcerative colitis.

13. *In vitro* use, according to claim 12, of the proteins IL1B, calprotectin and SIRT1, or of the transcripts encoding thereof, or of a kit comprising reagents for the identification of the proteins IL1B, calprotectin and SIRT1, or of the transcripts encoding thereof.

14. *In vitro* use of biomarker signatures identified by following the method of claims 1, 2 and 7, for the diagnosis or screening of a chronic inflammatory disease selected from the group comprises: IBD, arthritis or psoriasis, wherein the IBD is preferably selected from Chron's disease or ulcerative colitis.

15. Anti-inflammatory drugs selected from the group comprising:
a) Salicylates;
b) Immunosuppressants selected from azathioprine, methotrexate and mercaptopurine;
c) Biological treatments selected from adalimumab, golimumab, infliximab, risankisumab, upadicitinib, ustekinumab and vedolizumab;
d) Corticosteroids selected from: prednisone, cortisone, betamethasone, budesonide and beclomethasone;
for use in the treatment of a chronic inflammatory disease selected from the group comprising: IBD, preferably selected from Chron's disease or ulcerative colitis, arthritis or psoriasis, wherein the method comprises diagnosing patients that suffer from the chronic inflammatory disease by i) using the signatures identified by following the method of claims 1, 2 or 7, or ii) following the method of claims 3 to 7.
